# EUROPÄISCHE PATENTSCHRIFT

(11) **EP 1 549 372 B1**
(45) Veröffentlichungstag und Bekanntmachung des Hinweises auf die Patenterteilung: **05.01.2011**
(21) Anmeldenummer: 03798893.8
(22) Anmeldetag: 05.09.2003
(51) Int. Cl.: A61M 15/00

(54) **PULVERINHALATOR**
POWDER INHALER
INHALATEUR DE POUDRE

(30) Priorität: 26.09.2002 DE 10244795
(43) Veröffentlichungstag der Anmeldung: 06.07.2005
(73) Patentinhaber: Boehringer Ingelheim Pharma GmbH & Co.KG, 55216 Ingelheim am Rhein (DE)
(72) Erfinder: HOCHRAINER, Dieter, 57392 Oberkirchen (DE); SCHIEWE, Jörg, 55129 Mainz (DE); ZIERENBERG, Bernd, 55411 Bingen am Rhein (DE); DUNNE, Stephen, Stowmarket, Suffolk IP14 3AE (GB)
(74) Vertreter: Simon, Elke Anna Maria
(86) Internationale Anmeldenummer: PCT/EP2003/009850
(87) Internationale Veröffentlichungsnummer: WO 2004/030734

(56) Entgegenhaltungen:
- WO-A-90/07351
- WO-A-92/04066
- WO-A-92/04928
- US-A- 4 702 415
- US-A- 5 546 932

## Beschreibung

Die Erfindung betrifft einen Pulverinhalator gemäß dem Oberbegriff des Anspruchs 1.

Ein derartiger Pulverinhalator ist aus der WO-A-90/07351 bekannt.

Derartige Pulverinhalatoren werden für die Bereitstellung inhalierfähiger Arzneistoffe benötigt. Insbesondere bei Erkrankungen im Lungen- und Bronchialbereich werden die Arzneistoffe als inhalierfähige Arzneistoffe (Inhalativa) benötigt und zur Verfügung gestellt.

Im Rahmen der vorliegenden Erfindung ist unter dem Begriff "Arzneistoff" der wirksame Bestandteil eines Arzneimittels zu verstehen, der üblicherweise auch als Pharmakon oder Wirkstoff bezeichnet wird.

Der Begriff "Arzneistoff-Formulierung" umfaßt grundsätzlich neben Pulver-Formulierungen auch Lösungs-Formulierungen und Suspensions-Formulierungen. Für jede Art von Formulierung wurden verschiedene Inhalationssysteme entwickelt. Die Lösungs- oder Suspensionsformulierungen werden in einem pharmakologisch geeigneten Lösungsmittel formuliert. Als Lösungsmittel kommen grundsätzlich zum Beispiel Wasser oder verflüssigte Treibgase zum Einsatz. Bevorzugt wurden in der Vergangenheit Fluorchlorkohlenwasserstoffe, neuerdings Fluorkohlenwasserstoffe, verwendet. Im Fall von derartigen mit leicht flüchtigen Treibgasen betriebenen Inhalatoren wird das Arzneimittel ebenfalls als Lösung oder Suspension in dem Treibmittel formuliert.

Im Rahmen der vorliegenden Erfindung stehen die Pulver-Formulierung bzw. der zu ihrer Dispergierung notwendige Pulverinhalator im Vordergrund. Es sei aber an dieser Stelle darauf hingewiesen, dass die drei unterschiedlichen Arzneistoff-Formulierungen mit ihren speziellen Eigenschaften ganz unterschiedliche Anforderungen an den Inhalator stellen.

Üblicherweise werden in Inhalatoren die Formulierungen in einem Reservoir gelagert bzw. bevorratet, weshalb es notwendig ist, daß die verwendeten Formulierungen eine ausreichende Lagerstabilität aufweisen. Hierzu können dem Arzneistoff Hilfsstoffe zugesetzt werden, mit denen die physiko-chemischen Eigenschaften eingestellt werden, die die qualitätsbestimmenden Parameter wie Verfügbarkeit und Haltbarkeit in gewünschter Weise beeinflussen.

Die in einem Pulverinhalator bevorratete Arzneistoff-Formulierung wird zerstäubt und als Aerosol vom Patienten eingeatmet. Dabei wird der Arzneistoffe in inhalierfähiger Form bereitgestellt.

Normalerweise wird jedoch bei diesem Vorgang nicht die gesamte **abgemessene Dosis** als Aerosol ausgebracht, sondern nur ein Teil davon. Das liegt daran, dass ein Teil der Pulverformulierung im Vorratsgefäß zurückbleibt, nur aufgewirbelt wird und sich dann an einer anderen Stellen des Inhalators wieder abscheidet.

Den Teil der abgemessenen Dosis, der das Mundstück des Pulverinhalators verläßt wird als **ausgebrachte Dosis** bezeichnet.
Pulverteilchen können beim Inhaliervorgang nur dann in die Lunge gelangen, wenn der aerodynamischer Partikeldurchmesser kleiner als 5 µm ist. Die Konsequenz ist, das nur ein Teil der ausgebrachten Dosis auch tatsächlich in die Lunge gelangen kann. Dieser Anteil kann nur durch aufwendige Versuche am Patienten bestimmt werden. Aus diesem Grund wurden in-vitro Tests entwickelt, mit denen in einem einfachen Laborexperiment der **aerodynamische Feinanteil** ermittelt wird, der mit dem lungengängigen Teil der ausgebrachten Dosis korreliert. Der aerodynamische Feinanteil ist definiert als der Anteil der abgemessenen Dosis in Prozent, der einen aerodynamischen Partikeldurchmesser kleiner als 5,8 µm besitzt.

Im Rahmen der vorliegenden Erfindung ist unter dem aerodynamischen Partikeldurchmesser der Teilchendurchmesser zu verstehen, der dem Äquivalentdurchmesser einer Kugel der Dichte von 1 g/cm³ entspricht, die die gleiche Sedimentaionsgeschwindigkeit in Luft besitzt wie das untersuchte Teilchen.

Um einen möglichst hohen aerodynamischen Feinpartikelanteilzu erzielen, sind folgende Überlegungen zielführend.

Zunächst muß eine Pulverformulierung bereitgestellt werden, die den Arzneistoff in mikronisierter Form enthält. Der überwiegende Teil aller Arzneistoffpartikel sollte eine Größe im Bereich von 1 - 5 µm besitzen. Da mikronisierte Pulver als Haufwerke eine hohe Tendenz zur Ausbildung von Partikelagglomeraten zeigen, enthält die Pulverformulierung meist Hilfsstoffe, die die Deagglomeation der mikronisierten Arzneistoffpartikel erleichtern und auch die Fließfähigkeit erhöhen. Ein weiterer qualitätsrelevanter Parameter für die Pulverformulierung ist deren chemische und physikalische Stabilität. Chemische Stabilität ist gewährleistet, wenn der Arzneistoff sich bei der Lagerung nicht in Zersetzungsprodukte umwandelt. Physikalische Stabilität bezieht sich darauf, daß sich der gemessene aerodynamische Feinanteil über die Lagerzeit nicht verändert.

Ein geeigneter Pulverinhalator muß eine definierte Menge der Pulverformulierung, die abgemessene Dosis, während des Inhaliervorgangs des Patienten in ein Aerosol überführen, wobei möglichst hohe Werte für die ausgebrachte Dosis und den aerodynamischen Feinpartikelanteil erreicht werden sollten. Um das zu erreichen, ist eine wichtige Funktion des Pulverinhalators, die im Haufwerk der Pulverformulierung vorhandenen Partikelagglomerate des Arzneistoffs möglichst effizient zu Deagglomerieren, da größere Teilchen beim Einatmen bereits im Mund- und Rachenraum abgeschieden werden und nur Teilchen mit aerodynamischen Partikeldurchmessern kleiner als 5 µm in die Lunge gelangen. Damit ergibt sich eine mehr oder weniger große Differenz zwischen dem Anteil der ausgebrachten Dosis bezogen auf die abgemessene Dosis und dem aerodynamischen Feinpartikelanteil, der maßgeblich durch die Effizienz der Zerstäubung beeinflußt wird.

Vor dem Hintergrund der gemachten Ausführungen muß die Teilchengröße in engen Grenzen reproduzierbar sein, damit Schwankungen der ausgebrachten Dosis und des aerodynamischen Feinanteils vermieden werden. Bei jeder Betätigung des Inhalators soll eine annähernd gleichgroße Menge Arzneistoff verabreicht werden, wobei die ausgebrachten Dosen über annähend gleiche Größenverteilungen der Arzneistoffpartikel verfügen sollen.

Aber auch unter dem Gesichtspunkt der Effizienz und einem möglichst ökonomischen Umgang mit Arzneistoffen wird angestrebt, einen möglichst großen aerodynamischen Feinpartikelanteil - wie oben definiert - zu erzeugen.

Nach dem Stand der Technik kommen grundsätzlich zwei verschiedene Systeme von Pulverinhalatoren zum Einsatz.

Die sogenannten **passiven Inhalatoren** nutzen in der Regel die eingeatmete Luft des Patienten zur Zerstäubung der Pulver-Formulierung ohne zusätzliche Hilfsenergiequellen - beispielsweise in Form von Druckluft - einzusetzen. Diese Pulverinhalatoren sind in der Art konzipiert, daß Pulver entweder in Form einer Einzeldosis (premetered dose) zum Beispiel in einer vorgefertigten Kapsel enthalten ist oder auch mehrere vordosierte Mengen in einem Mehrdosis-Behältnis, das in den Inhalator eingesetzt wird, bereitgestellt werden. Bei Gebrauch wird die Kapsel bzw. eines der Mehrdosisbehältnisse aufgestochen, wobei die Entleerung und Zerstäubung des Pulvers mittels der eingeatmeten Luft des Patienten erfolgt.

Das Pulver kann auch als Pulvervorrat (bulk powder) im Inhalator vorliegen, wobei eine einzelne Dosis über eine Dosiervorrichtung bereitgestellt wird bevor sie über die Atemluft des Patienten aus dem Inhalator heraus transportiert wird.

Es ist offensichtlich, daß bei den beschriebenen Pulverinhalatoren der aerodynamische Feinpartikelanteil stark von dem Atemmanöver des Patienten abhängig ist.

Vor dem Hintergrund der gemachten Ausführungen ist man dazu übergegangen sogenannte **aktive Pulverinhalatoren** einzusetzen, weiche über gespeicherte Energie, z.B. Druckgas verfügen. Durch Nutzung des Druckgases zum definierten Ausbringen und Zerstäuben der Pulver-Formulierung wird die Unabhängigkeit vom Atemmanöver des Patienten erreicht.

Um Deagglomeration und effiziente Zerstäubung zu bewirken und die gewünschte Teilchengröße und Teilchengrößenverteilung zu realisieren, wurden nach dem Stand der Technik im wesentlichen zwei Wege beschritten.

Bei einigen in der Literatur beschriebenen Inhalatoren wird die Deagglomeration des Pulvers durch Aufprall der Teilchen auf sogenannte Prallflächen unterstützt. Beispielsweise mit Hilfe von Druck werden die Pulverpartikeln gezielt auf diese Prallflächen gelenkt, um eine Deagglomation der Partikel zu erzielen. Dabei stellt sich aber der Effekt ein, daß ein Teil der auf die Prallfläche auftreffenden Pulverpartikel an der Prallfläche haften bleiben und hier deponiert werden. Das hat zur Folge, daß eine möglichst genaue und reproduzierbare Dosierung nicht möglich ist.

Nachteilig ist, daß bei der Verwendung von unter Druck stehenden Gasen zur Deagglomeration von Pulverformulierungen sehr hohe Aerosolgeschwindigkeiten erzeugt werden. Sehr hohe Aerosolgeschwindigkeiten wiederum führen dazu, daß der Anteil der Dosis, der die Lunge erreicht, verkleinert wird. Daher wurden für Pulverinhalatoren, die mit Druckgasen arbeiten, zusätzliche Spacer/ Separatoren vorgesehen, die die Aufgabe haben, die Geschwindigkeit der gebildeten Aerosolpartikel herabzusetzen.

Diese Spacer/ Separatoren (oder Deagglomerationskammern), in denen die Geschwindigkeit der Pulverteilchen abgebremst wird, werden vor dem Mundstück des Inhalators angeordnet und machen den Inhalator sperrig und unhandlich. Inhalatoren für den pharmazeutischen Bereich sollen aber klein und handlich sein, damit der Patient den Inhalator ständig mit sich führen kann.

Vor diesem Hintergrund ist es die Aufgabe der vorliegenden Erfindung, einen Pulverinhalator zur Dispergierung von pharmazeutischen Pulver-Formulierungen bereitzustellen, mit weichem die aus dem Stand der Technik bekannten Probleme der herkömmlichen Pulverinhalatoren eliminiert, zumindest aber gemindert werden und mit dem insbesondere ein hoher Anteil an Feststoffpartikeln kleiner als 5,8 µm erzeugt wird.

Gelöst wird diese Aufgabe durch einen Pulverinhalator der gattungsbildenen Art, der dadurch gekennzeichnet ist, daß in dem Inhalator eine Düse vorgesehen ist, durch welche das Aerosol vor Verlassen des Inhalators strömt, wie er im Anspruch 1 definiert ist.

Bei dem erfindungsgemäßen Pulverinhalator trägt das Druckmedium das Pulver aus der Pulverbevorratung und transportiert es durch die Düse.

Dabei wird das gasförmige Druckmedium stärker beschleunigt und die in ihm enthaltenen Pulverpartikel werden von diesem mitgerissen, wodurch die Deagglomeration der Pulverpartikel erfolgt. Die vom gasförmigen Druckmedium infolge der höheren Geschwindigkeiten der Gasmoleküle auf die Partikel ausgeübten Scherkräfte bewirken dabei ein Aufbrechen der Partikelagglomerate.

In der einfachsten Ausführungsform der Düse handelt es sich um eine sogenannte Lochblende. Die Deagglomerations-Effizienz wurde anhand der aerodynamischen Größenverteilung der Partikel mit Hilfe eines Kaskadenimpaktors gemessen. Als entscheidende Meßgröße wurde der Feinpartikelanteil als Prozentsatz der vordosierten Pulvermenge bestimmt. Dazu wurde jeweils 5 mg mikronisiertes Fenoterol HBr als Arzneistoff verwendet. Im folgenden ist für diese Düsenvariante der gemessene Feinpartikelanteil (Fine Particle Fraction) angegeben.

**Tabelle 1**

| **Düse** | **Durchmesser [mm]** | **Druckluft** | | **Feinpartikelanteil [%]** |
|---|---|---|---|---|
| | | **Volumen [ml]** | **Druck [bar]** | |
| | | | | |
| Lochblende | 0,4 | 12 | 4 | 44 |
| Lochblende | 0,6 | 7,5 | 4 | 34 |
| Lochblende | 0,8 | 12 | 0,5 | 42 |

Als Referenz diente ein Inhalator der Marke HandiHaler®. Ein solches Gerät wird beispielsweise in der EP 0911047 beschrieben. Dieser Inhalator (Handihaler) ist für die Inhalation pulverförmiger Arzneimittel aus Kapseln bestimmt. Er ist gekennzeichnet durch ein Gehäuse, enthaltend zwei Fenster, ein Deck, in dem sich Lufteinlaßöffnungen befinden und welches mit einem über ein Siebgehäuse befestigten Sieb versehen ist, eine mit dem Deck verbundene Inhalationskammer, an der ein mit zwei geschliffenen Nadeln versehener, gegen eine Feder beweglicher Drücker vorgesehen ist sowie ein über eine Achse klappbar mit dem Gehäuse, dem Deck und einer Kappe verbundenes Mundstück. Dieses Gerät liefert bei einer Luftmenge von 4l und einem Druckabfall von 4 kPa einen Feinpartikelanteil von 18-20% einer vordosierten Menge von 5 mg Fenoterol HBr. Damit zeigen die Werte nach *Tabelle 1 eine deutlich höhere Deagglomerationseffizienz des erfindungsgemäßen* beschriebenen Systems.

Dient als Düse eine Lochblende, werden Löcher mit einem Durchmesser von 0,1 bis 3 mm, vorzugsweise 0,3 bis 2 mm, insbesondere 0,5 bis 1,5 mm bevorzugt.

In einer anderen Ausführungsform des Pulverinhalators handelt es sich bei der Düse nicht lediglich um eine einfache Lochblende, sondern die Blende weist einen sich verjüngenden Eintrittsabschnitt auf. Diese Düse kann dahingehend weiterentwickelt werden, dass auch der Austrittsabschnitt sich erweitert, so dass die Düse die Form einer sich in der Mitte verjüngenden Röhre aufweist, bei der die Winkel der durch die Abschnürung entstandenen sich verjüngenden Teilröhren gleich oder unterschiedlich sein können. Ein sich kontinuierlich verjüngender Eintrittsabschnitt bzw. erweiternder Austrittsabschnitt gewährleistet eine stetige Beschleunigung bzw. Abbremsung des Aerosolstroms. Die Strömung liegt laminar an der Innenwand der Düse an und eine Ausbildung von turbulenten Zonen und sogenannten "Totwassergebieten", in denen die Aerosol-Strömung steht und nicht mehr strömt, die Strömung also gewissermaßen zum Erliegen kommt, wird unterbunden. Die Vermeidung von "Totwassergebieten" ist insbesondere im Hinblick auf die Ablagerung von Pulverteilchen als vorteilhaft anzusehen.

Bei Düsen mit konvergierendem Eintrittsabschnitt und lochförmigem Austritt, also ohne divergierenden Austritt, werden Löcher mit einem Durchmesser von 0,1 bis 3 mm, vorzugsweise 0,3 bis 2 mm, insbesondere 0,5 bis 1,5 mm bevorzugt.

Bei Düsen mit konvergierendem Eintrittsabschnitt und divergierendem Austrittsabschnitt werden engste Querschnitte mit einem Durchmesser von 0,1 bis 3 mm, vorzugsweise 0,3 bis 2 mm, insbesondere 0,5 bis 1,5 mm bevorzugt.

Bei der Ausbildung der Düse mit konvergierndem Eintrittsabschnitt und divergierendem Austrittsabschnitt hat die Eintrittsöffnung der Düse einen Durchmesser von bevorzugt 2 bis 6 mm, insbesondere 3 bis 5 mm. Der Eintrittsabschnitt hat einen konkaven oder linearen Verlauf mit einem Öffungswinkel von bevorzugt 10 bis 50°. Der Eintrittsabschnitt weist bevorzugt eine Länge von 3 bis 10 mm, bevorzugt 5 bis 8 mm auf.

Bei Düsen mit divergierendem Austritt weist die Öffnung des Austrittsabschnittes einen Durchmesser von bevorzugt 0,1 bis 10 mm, besonders bevorzugt 0,3 bis 7,5 mm, insbesondere bevorzugt von 0,4 bis 5 mm auf. Der Öffnungswinkel des Austrittsabschnittes beträgt 7 bis 15°, bevorzugt 8 bis 12°.Die Form des Austrittsabschnittes kann aber auch von einer linearen Divergenz, gefolgt von einem rohrförmigen Abschnitt geprägt sein. Seine Länge beträgt bevorzugt 3 bis 50 mm, besonders bevorzugt 5 bis 15 mm.

Vorteilhaft sind dabei insbesondere Ausführungsformen des Pulverinhalators, bei denen die Düse eine Lavaldüse ist.

Bei dieser Ausführungsform des Pulverinhalators durchströmt das Aerosol die Düse, wobei es zunächst in den sich verjüngenden Eintrittsabschnitt eintritt, das Mittelstück, in dem der engste Querschnitt der Düse angeordnet ist, passiert und anschließend den sich erweiternden Austrittsabschnitt durchströmt.

Dabei wird das Aerosol in dem sich verjüngenden Eintrittsabschnitt beschleunigt, wobei wieder das gasförmige Druckmedium stärker beschleunigt wird und die in ihm enthaltenen Pulverpartikel mitreißt, wodurch der Transport der Pulverpartikel erfolgt. Die vom gasförmigen Druckmedium infolge der höheren Geschwindigkeiten der Gasmoleküle auf die Partikel ausgeübten Scherkräfte bewirken ein Aufbrechen der Partikelaggregate/ - agglomerate in kleinere Partikel von geringerem Durchmesser, wodurch eine Deagglomeration erzielt wird. Die höchste Geschwindigkeit erreicht das Aerosol im oder nach dem engsten Querschnitt der Düse, welcher sich im Mittelstück befindet.

Je nach dem anliegenden Druck werden in der Laval-Düse Gasgeschwindigkeiten unterhalb oder oberhalb der Schallgeschwindigkeit erzeugt.

Die Gasgeschwindigkeit im Verhältnis zur Schallgeschwindigkeit wird als Mach-Zahl (Ma) bezeichnet. Die **Machzahl** berechnet sich als Quotient aus Gasgeschwindigkeit und Schallgeschwindigkeit. Somit ist Ma = 1, wenn die erreichte Gasgeschwindigkeit die Größe der Schallgeschwindigkeit erreicht hat. Von Überschallgeschwindigkeit spricht man, wenn gilt Ma > 1.

Vorteilhaft sind Ausführungsformen der Düse in der Art, daß im Austrittsabschnitt das Druckmedium auf Überschallgeschwindigkeit beschleunigt wird.

Dies setzt voraus, daß die Aerosol-Strömung im engsten Querschnitt der Düse bereits Schallgeschwindigkeit erreicht. In diesem Fall, in dem das Aerosol im Mittelstück Schallgeschwindigkeit erreicht, d.h. Ma = 1, wird die Aerosolströmung im sich erweiternden Austrittsabschnitt der Düse zusätzlich auf Überschallgeschwindigkeit beschleunigt.

Die sich tatsächlich im sich erweiternden Austrittsabschnitt der Düse einstellende Geschwindigkeit - d.h. Ma-Zahl - hängt maßgeblich vom stromaufwärts der Düse bereitgestellten Druck ab. Die Pulverpartikel werden dabei nicht wie die Gasmoleküle auf Überschallgeschwindigkeit beschleunigt, so daß sich ein Unterschied in der Geschwindigkeit ergibt, der wiederum dazu führt, daß Scherkräfte auf die Pulverpartikel ausgeübt und diese deagglomeriert werden.

Bei dieser Ausführungsform kommt es im Bereich des sich erweiternden Austrittsabschnitts zur Ausbildung eines sogenannten Mach-Schocks. Beim Durchtritt durch diesen Mach-Schock werden die Gasmoleküle innerhalb einer kurzen Strecke von wenigen Millimetern stark auf Unterschallgeschwindigkeit verzögert. Die Pulverpartikel, die vor Durchtritt durch den Mach-Schock langsamer als die Gasmoleküle waren, werden weniger stark verzögert als die Gasmoleküle, so daß nach dem Machschock die Pulverpartikel eine höhere Geschwindigkeit aufweisen als die Gasmoleküle. Hierdurch kommt es zu extrem hohen Reibungs- bzw. Scherkräften, welche an den Pulverteilchen angreifen. Die Folge ist eine weitere Deagglomeration der Partikel, was zu einer noch besseren Inhalierfähigkeit aufgrund der sich einstellenden Teilchengrößen und Teilchengrößenverteilung führt.
Im folgenden ist für einige Laval-Düsenvarianten der gemessene Feinpartikelanteil (Fine Particle Fraction) für 5 mg Fenoterol HBr, mikronisiert angegeben.

**Tabelle 2**

| **Düse** | **Durchmesser [mm]** | **Druckluft** | | **Feinpartikelanteil [%]** |
|---|---|---|---|---|
| | | **Volumen [ml]** | **Druck [bar]** | |
| | | | | |
| Lavaldüse | 0,5 | 7,5 | 4 | 30 |
| Lavaldüse | 0,8 | 7,5 | 4 | 26 |
| Lavaldüse | 1,5 | 7,5 | 4 | 32 |

### Referenzwerte: s. Tabelle 1

Da eine zu hohe Strömungsgeschwindigkeit den Feinpartikelanteil, der während des Inhalationsvorgangs die Lunge des Patienten erreichen kann, verringert, sollte die Geschwindigkeit am Austritt des Mundstückes nicht größer als 20 m/s, vorzugsweise nicht größer als 10m/s, insbesondere nicht größer als 5 m/s sein.

Da zur Deagglomeration des Pulvers beim Durchgang durch eine Düse hohe Strömungsgeschwindigkeiten erzeugt werden müssen, ist es generell notwendig, die Aerosolgeschwindigkeit auf der Wegstrecke zwischen Düse und Mundstück zu reduzieren. Dies läßt sich auf verschiedene Art und Weise bewerkstelligen, so daß auf die Verwendung des erfindungsgemäßen Pulverinhalators zusammen mit einem Spacer verzichtet werden kann. Im einzelnen ergibt sich bevorzugt folgendes:

Das am Düsenauslaß austretende Aerosol kann auf verschiedene Weise mit der Atemluft des Patienten auf der Wegstrecke zwischen Düse und Mundstück vermischt werden.

Die Erzeugung einer wirbelförmigen Strömung der Einatemluft des Patienten ist eine dafür geeignete Maßnahme. Die durch die Düse tretende Aerosolwolke wird in den Wirbel der Einatemluft injiziert, wodurch sich die Vorwärtskomponente der Geschwindigkeit der Aerosolwolke reduziert.

Eine Auslegung der Düse in der Art, dass die Einatemluft und die aus der Düse austretende Aerosolströmung entgegengerichtet sind, wodurch ebenfalls eine Verzögerung erzielt werden kann, stellt eine andere Maßnahme zur Verringerung der Strömungsgeschwindigkeit der Aerosolwolke dar.

In einer alternativen Ausführungsform befindet sich die Eintrittsöffnung der Einatemluft windschief oder senkrecht zur Strömungsrichtung der Düse.

Im folgenden ist für zwei verschiedene Maßnahmen und verschiedene Düsen die gemessene Aerosolgeschwindigkeit des Feinanteils in einem Abstand von 10 cm hinter der Düse angegeben (Tabelle 3)

**Tabelle 3**

| **Düse** | **Durchmesser [mm]** | **Druckluft** | | **Aerosolgeschwindigkeit [m/s]** |
|---|---|---|---|---|
| | | **Volumen [ml]** | **Druck [bar]** | |
| | | | | |
| Lochblende | 0,8 | 2,5 | 4 | 19 |
| Lochblende | 0,6 | 6,7 | 1,5 | 12 |
| Lochblende | 0,6* | 5,4 | 1,5 | 5 |
| Lochblende | 0,6** | 5,4 | 1,5 | 2 |
| Lochblende | 0,4 | 16,7 | 0,5 | 3 |
| Lavaldüse | 0,5 | 5,4 | 1,5 | 2 |

| | | | | |
|---|---|---|---|---|
| * Erzeugung einer wirbelförmigen Strömung der Einatemluft des Patienten ** Einatemluft und die aus der Düse austretende Aerosolströmung sind entgegengerichtet | | | | |

Um die Geschwindigkeit der aus der Düse austretenden Aerosolwolke im Inhalator zu reduzieren*,* hat es sich als vorteilhaft erwiesen, diese Aerosolwolke mit einem entgegenkommenden Luftrom zu vermischen. Vorteilhaft ist es dabei, wenn dieser entgegenkommende Luftstrom durch den Einatmungsvorgang des Benutzers erzeugt wird. Üblicherweise wird bei einem Pulverinhalator dafür gesorgt, dass der Patient beim Einatmen der Pulverwolke auch Luft mit einatmet, um einen komplikationslosen Einatmungsvorgang gewährleisten zu können. Dafür können z.B. im Mundstück Luftschlitze ausgebildet sein, durch die der Patient automatisch beim Einatmen der Partikelwolke auch Luft mit einatmet. Im Rahmen des erfindungsgemäßen Pulverinhalators können diese Lufteinlassöffnungen so ausgebildet sein, dass die einströmende Luft der Aerosolwolke entgegenläuft entweder als rein entgegenlaufender oder auf die Aerososwolke aufprallender Luftstrom, der so deren Geschwindigkeit abbremst. Konstruktiv kann diese Aufgabe dadurch gelöst werden, dass z.B. das Mundstück senkrecht zu dem Weg steht, den die Aerosolwolke beim Austreten aus der Düsenöffnung nimmt. Die Lufteinlassschlitze sind dann ebenfalls senkrecht zum Mundstück und in einer Linie mit dem Austrittsweg der Aerosolwolke aus der Düse, aber der Düse genau entgegengesetzt ausgebildet. Eine solche Konstruktion hätte vereinfacht ausgedrückt die Form eines T-Stückes, wobei die Düse für die Aerosolwolke und die Lufteinlässe für die Gegenluft jeweils die Endpunkte des T-Balkens bestimmen und das Mundstück dem Fußpunkt des T entspricht.

In einer alternativen Ausführungsform münden die Düse und die Lufteinlasslöcher in einer Wirbelkammer, werden darin miteinander verwirbelt und treten dann durch ein Mundstück aus.

Eine solche Wirbelkammer kann im einfachsten Fall ein hohler Raum sein, in den an einer Stelle die Düse einmündet: Die Lufteinlässe können dann genau gegenüber der Düse oder bevorzugt senkrecht dazu oder zumindest windschief versetzt in den Raum münden. Der Raum hat dann einen Ausgang zum Mundstück. Dabei können eine gedachte aus der Düsenöffnung heraustretende Linie und eine aus dem Mundstück in den Raum eintretende Linie eine Gerade bilden oder sie treffen sich in einem Winkel oder sie liegen colinear zueinander.

Im einfachsten Fall besteht der erfindungsgemäße Pulverinhalator aus einem Gehäuse mit einem Mundstück. Dem Mundstück vorgelagert, im Inneren des Gehäuses befindet sich die oben beschriebene Düse. Weiter im Inneren befindet sich ein Raum zur Aufnahme der Pulverformulierung, die zerstäubt werden soll.

Bevorzugt weist der Pulverinhalator ein Drucksystem auf, welches ein unter Druck stehendes Gas auf die zu dispergierende Menge der Pulverformulierung leitet, so dass diese dispergiert wird und das entstehende Aerosol durch das oben beschriebene Düsensystem unter Aufbrechen der gröberen Teilchen in das Mundstück geleitet wird. Der Pulverinhalator weist dabei in seinem Inneren Kanäle auf, die den Weg des unter Druck abgebenen Gases durch den Inhalator bis in das Mundstück vorgeben. In dem Raum zur Aufnahme der zur Dispergierung vorgesehenen Pulverformulierung kann das Pulver lose liegen oder es befindet sich in einem Container, z.B. in einer Kapsel oder einem Blister, der vor Durchleiten des Druckgases so geöffnet wird, dass das Druckgas das Pulver weitgehend ohne Rückstände mitreißen kann. Im Fall eines Einweginhalators weist dieser keine weitere Bevorratung für weitere Dosierungen der Puvlerformuleirungen auf. Im Fall eines Geräts zur mehrmaligen Benutzung kann der Inhalator ein oder mehrere Reservoir(e) für die Pulverformulierung aufweisen. So kann das Reservoir lediglich ein Raum sein, der das lose Pulvergemisch beinhaltet und von dort werden abgemessene Mengen in den Zerstäubungsraum überführt. Alternativ kann das Reservoir eine Ansammlung von Kapseln sein, die mit der Pulverformulierung gefüllt sind und mechanisch oder manuell in die Zerstäubungskammer eingebracht werden. Schließlich kann es sich bei dem Reservoir auch um einen Blister mit vielen Taschen für die Pulverformulierung handeln, wobei dann immer eine dieser Taschen in den Zerstäubungssraum eingebracht wird. Derartige Reservoirsysteme und die Überführung der Pulverformulierung aus dem Reservoir in den Vernebelungsraum sind aus dem Stand der Technik bekannt, weshalb an dieser Stelle nicht näher darauf eingegangen werden soll.

Als Druckmediumsystem kann der erfindungsgemäße Pulverinhalator eine Druckluftpatrone aufweisen, eine mit einem anderen Gas als Luft gefüllte Patrone, z.B. Stickstoff, Kohlendioxid, ein Edelgas, wie Helium oder Argon, oder sogar ein Fluorkohlenwasserstoff, ein Alkan und dergleichen. Vorteilhaft sind Ausführungsformen des Pulverinhalators, bei denen das Druckmediumsystem ein System ist, welches Luft aus der Umgebung ansaugt und dann die Luft gezielt unter Kompression und Druck in Richtung der zu vernebelnden Formulierung abgibt. Zum einen ist Luft als Trägermedium für die Pulverpartikel das wohl unbedenklichste Medium für den Patienten. Zum anderen ist es leicht verfügbar. Die bevorzugte Ausführungsform des Pufverinhalators entnimmt der Umgebung die benötigte Menge an Luft, komprimiert sie und nutzt sie dann als Trägermedium für die Pulver-Formulierung. Ein Auswechseln des Druckmediumsystem, wie dies der Fall bei einem in einer Patrone gespeicherten Druckmedium der Fall wäre, entfällt.

Vorteilhaft können aber auch Ausführungsformen des Pulverinhalators sein, bei denen das Druckmediumsystem eine Patrone umfaßt, welche ein unter Druck stehendes Druckmedium bevorratet. Im Gegensatz zu dem zuvor beschriebenen Pulverinhalator, ist diese Ausführungsform weniger komplex in ihrem Aufbau und daher kostengünstiger und in ihren Abmessungen kleiner.

In jedem Fall ist das Druckmediumsystem derart, dass der Verwender des Inhalators gezielt einen Ausstoss des Druckmediums erzeugen kann.

Vorteilhaft sind Ausführungsformen des Pulverinhalators, die dadurch gekennzeichnet sind, daß die Vorrichtung zur Bevorratung der Pulver-Formulierung zwischen dem Druckmediumsystem und Düse angeordnet ist in der Art, daß das Druckmedium die Vorrichtung passieren muß, wobei vorzugsweise die Vorrichtung zur Bevorratung der Pulver-Formulierung eine mit Pulver gefüllte Kapsel aufweist. Bevorzugte Ausführungsformen des Pulverinhalators sind die, bei denen die Kapsel als Verbrauchsmaterial austauschbar ist. Hierbei wird ein Mechanismus am Pulverinhalator vorgesehen, mit dem ein Austausch der Kapsel erfolgen kann.

Das Druckmedium durchströmt bei dieser Ausführungsform die Vorrichtung zur Bevorratung der Pulver-Formulierung und verteilt bzw. nimmt das Pulver in sich auf, so daß nach Durchströmen der Bevorratungskammer das gewünschte Aerosol vorliegt.

Vorteilhaft sind Ausführungsformen des Pulverinhalators, bei denen die Vorrichtung zur Bevorratung der Pulver-Formulierung ein Mehrdosis-Blister-Behältnis umfaßt. Dabei können solche Mehrdosis-Blister-Behältnisse linear als Blisterband, flächig als Blister-Scheiben oder Blister-Ringe oder dreidimensional als zylindrische oder vielflächige Körper ausgeführt sein. Derartige Mehrdosissysteme können 2 bis 90 Dosen enthalten, bevorzugt 5 bis 60 Dosen, insbesondere 7 bis 30 Dosen, wobei jede Dosis in einer separaten Tasche gelagert ist, die bei Gebrauch mittels einer geeigneten Vorrichtung geöffnet wird.

Wie bereits ausgeführt werden Pulverinhalatoren bevorzugt, bei denen Luft als Druckmedium vorgesehen wird.

Sofern das Druckmediumsystem nicht prinzipbedingt per Hand betätigt wird, sondern ein zu betätigendes Stellglied - beispielsweise in Form eines Aktuator-Ventils - aufweist, durch dessen Öffnen oder Schließen das Druckmedium freigegeben wird, sind Ausführungsformen des Pulverinhalators vorteilhaft, die im Mundstück ein Durchfluß-Sensor vorsehen, der den Einatemfluß des Patienten misst und ab einem bestimmten Wert ein Eingangssignal für das Druckmediumsystem bzw. dessen Stellglied generiert.

Der Durchflußsensor mißt den Luftstrom beim Einatmen des Patienten und generiert ein Eingangssignal, mit welchem das Stellglied beaufschlagt wird. Es öffnet und läßt das Druckmedium ausströmen, wenn die Durchflußrate in einem für die Inhalation geeigneten Bereich liegt, wobei das Stellglied das Druckmediumsystem nicht freigibt, falls diese Durchflußrate außerhalb des bevorzugten Bereiches liegt. Dieses automatische Öffnen und Schließen erspart das Vorsehen einer zusätzlichen Betätigungsvorrichtung und sorgt für mehr Bedienungsfreundlichkeit und eine leichtere Handhabung des Inhalators.

Der erfindungsgemäße Pulverinhaltor weist folgende Vorteile gegenüber dem Stand der Technik auf:
- aus einem in einem geeigneten Behältnis vordosierten Pulverhaufwerk wird ein Aerosol erzeugt, wobei die in Form eines unter Druck stehenden Gases vorhandene Energie zur Überwindung der Agglomerationskräfte der mikronisierten Pulverteilchen untereinander verwendet wird, so dass ein Aerosol mit vergleichsweise überdurchschnittlich hohen Anteil von Feststoffpartikeln kleiner als 5,8 µm erzeugt wird.
- das hier beschriebene System muss nicht auf fluorierten Treibgasen oder Fluor-ChlorKohlenwasserstoffen zurückgreifen.
- die Erzeugung des Aerosols und die erreichte Partikelgrößenverteilung ist unabhängig vom Atemmanöver des Patienten.
- die Pulverrückstände im Gerät sind konstruktionsbedingt minimal, da die Deagglomeration des Pulvers durch die Wirkung strömender Gase in einer Düse und nicht durch Impaktion an Prallflächen erreicht wird.
- der beschriebene Inhalator ist klein und kann durch den Patienten leicht mit sich geführt werden.
- Auf die Verwendung des Gerätes mit einem "Spacer" - wie für andere "aktive" Pulversysteme (Patent WO 99/6249) - kann verzichtet werden, da im Gerät durch entsprechende Vorkehrungen eine langsam fortbewegte Aerosolwolke erzeugt wird.

Im folgenden wird die Erfindung anhand mehrerer Ausführungsformen gemäß den beiden Zeichnungsfiguren näher erläutert. Hierbei zeigt:
- Fig.1: eine schematische Darstellung einer ersten Ausführungsform eines Pulverinhalators in der Seitenansicht, geschnitten,
- Fig.2: eine schematische Darstellung einer Düse einer zweiten Ausführungs- form eines Pulverinhalators in der Seitenansicht, geschnitten,
- Fig.3a: eine schematische Darstellung einer ersten Ausführungs- form eines Mehrdosis-Blister-Behältnisses zur Bevorratung der Pulver- Formulierung in der Seitenansicht,
- Fig.3b: eine schematische Darstellung einer zweiten Ausführungs- form eines Mehrdosis-Blister-Behältnisses zur Bevorratung der Pulver- Formulierung in der Seitenansicht,
- Fig.3c: eine schematische Darstellung einer dritten Ausführungs- form eines Mehrdosis-Blister-Behältnisses zur Bevorratung der Pulver- Formulierung in der Seitenansicht,
- Fig.3d: eine schematische Darstellung einer vierten Ausführungs- form eines Mehrdosis-Blister-Behältnisses zur Bevorratung der Pulver- Formulierung in der perspektivischen Darstellung,

- Fig.3e: eine schematische Darstellung einer fünften Ausführungs- form eines Mehrdosis-Blister-Behältnisses zur Bevorratung der Pulver- Formulierung in der perspektivischen Darstellung,
- Fig.4: eine schematische Darstellung einer Düse einer dritten Ausführungs- form eines Pulverinhalators in der Seitenansicht, geschnitten,
- Fig.5: eine schematische Darstellung einer Düse einer vierten Ausführungs- form eines Pulverinhalators in der Seitenansicht, geschnitten,
- Fig.6: eine schematische Darstellung einer ersten Ausführungs- form einer Vorrichtung zur Verzögerung der Aerosolströmung in der Seitenansicht, geschnitten, und
- Fig.7: eine schematische Darstellung einer zweiten Ausführungs- form einer Vorrichtung zur Verzögerung der Aerosolströmung in der Seitenansicht, geschnitten,

Nachfolgend sind gleiche Teile mit denselben Bezugszeichen versehen.

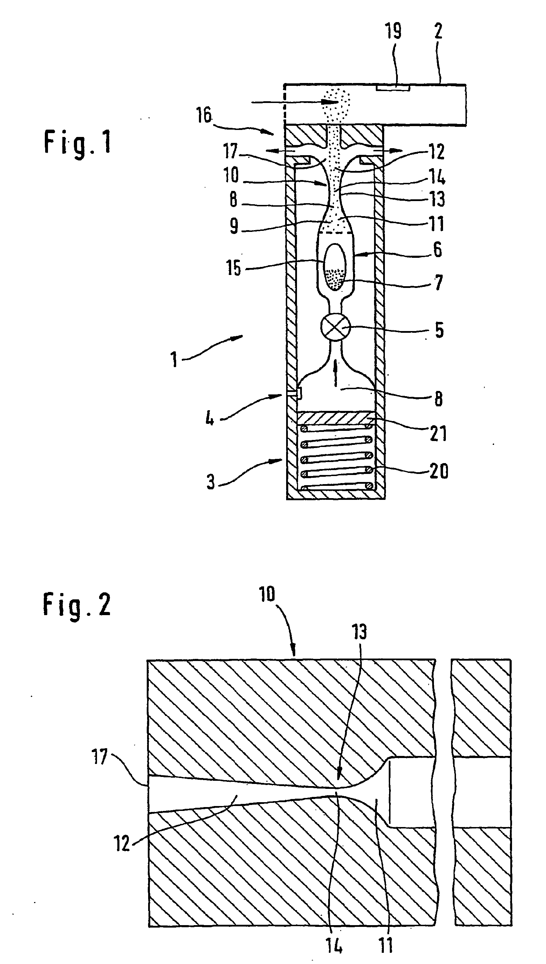

Figur 1 zeigt in einer schematische Darstellung eine erste Ausführungsform eines Pulverinhalators 1 zur Dispergierung von pharmazeutischen Arzneistoff Formulierungen in der Seitenansicht und geschnitten.

Der Pulverinhalator 1 verfügt an seinem oberen Ende über ein Mundstück 2. Er weist eine Hilfsenergiequelle in Form eines Druckmediumsystems 3 auf, wobei das Druckmediumsystems 3 mit einer Pumpe ausgestattet ist, die mit der Umgebung über ein Ventil 4 in Verbindung steht und die Umgebungsluft als Druckmedium 8 verwendet. Die Luft dient als Trägermedium für die Pulverpartikel 7 und wird von einem über eine Feder 20 kraftbeaufschlagten Kolben 21 während der Expansionsphase über das Ventil 4 angesaugt und beim Hochfahren des Kolbens komprimiert. Ein Auswechseln des Druckmediumsystem, wie dies der Fall bei einem in einer Patrone gespeicherten Druckmedium der Fall wäre, entfällt.

Das Druckmedium 8 verläßt das Druckmediumsystem 3 über ein Stellglied bzw. ein Aktuator-Ventil 5. Bei der in Figur 1 dargestellten Ausführungsform ist ein Durchflußsensor 19 im Mundstück 2 vorgesehen. Der Durchflußsensor 19 mißt den Luftstrom beim Einatmen des Patienten - Durchflußrate - und generiert ein Eingangssignal, mit welchem das Aktuator-Ventil 5 beaufschlagt wird. Es öffnet und läßt das Druckmedium 8 ausströmen, wenn die Durchflußrate in einem für die Inhalation geeigneten Bereich liegt, wobei das Stellglied 5 das Druckmediumsystem 3 schließt, falls diese Durchflußrate außerhalb dieses bevorzugten Bereiches liegt. Dieses automatische Öffnen und Schließen sorgt für mehr Bedienungsfreundlichkeit und optimiert die Bedingungen während die Inhalation.

Nach Austritt aus dem Druckmediumsystem 3 durchströmt das Druckmedium 8 eine Vorrichtung zur Bevorratung 6 der Pulver-Formulierung 7. Die Vorrichtung zur Bevorratung 6 der Pulver-Formulierung 7 verfügt über eine mit Pulver 7 gefüllte Kapsel 15. Der Mechanismus, mit dem ein Austausch der Kapsel erfolgen kann, ist nicht dargestellt.

Das Druckmedium 8 durchströmt bei dieser Ausführungsform die Vorrichtung zur Bevorratung 6 der Pulver-Formulierung 7 und nimmt einen Teil des Pulvers 7 in sich auf, so daß nach Durchströmen der Bevorratungskammer das gewünschte Aerosol 9 in der Art vorliegt, daß die Pulverpartikel 7 in dem gasförmigen Druckmedium 8 dispergiert sind.

Danach durchströmt das Aerosol 9 die Düse 10, wobei es zunächst in den sich verjüngenden Eintrittsabschnitt 11 eintritt, das Mittelstück 13, in dem der engste Querschnitt 14 der Düse 10 angeordnet ist, passiert und anschließend den sich erweiternden Austrittsabschnitt 12 durchströmt. Dieses Ausführungsbeispiel bedient sich also einer Laval-Düse.

Dabei wird das Aerosol 9 in dem sich verjüngenden Eintrittsabschnitt 11 beschleunigt, wobei das gasförmige Druckmedium 8 stärker beschleunigt wird und die in ihm enthaltenen Pulverpartikel 7 mitreißt, wodurch der Transport der Pulverpartikel 7 erfolgt. Die vom gasförmigen Druckmedium 8 infolge der höheren Geschwindigkeiten der Gasmoleküle 8 auf die Partikel 7 ausgeübten Scherkräfte bewirken ein Aufbrechen der Partikelaggregate/ - agglomerate 7 in kleinere Partikel von geringerem Durchmesser, wodurch eine Deagglomeration erzielt wird. Die höchste Geschwindigkeit erreicht das Aerosol 9 im oder nach dem engsten Querschnitt 14 der Düse 10, welcher sich im Mittelstück 13 befindet.

Figur 2 zeigt eine schematische Darstellung einer Düse 10 einer zweiten Ausführungsform eines Pulverinhalators in der Seitenansicht im Schnitt.

Das aufbereitete Aerosol, welches aus den in dem Druckmedium als Trägermedium dispergierten Pulverteilchen und dem Druckmedium selbst besteht, tritt in den sich kontinuierlich verjüngenden Eintrittsabschnitt 11 der Düse 10 ein, wird beschleunigt, passiert den engsten Querschnitt 14, der im Mittelstück 13 angeordnet ist, um danach den sich erweiternden Austrittsabschnitt 12 zu durchströmen und die Düse 10 durch den Austritt 17 zu verlassen.

Die in Fig. 2 dargestellte Lavaldüse 10 ist dadurch gekennzeichnet, daß sie über einen vergleichsweise langen Austrittsabschnitt 12 verfügt, der einen nur geringen Öffnungswinkel hier etwa 11°- aufweist.

Derartige Düsen 10 kommen bei Pulverinhalatoren zum Einsatz, bei denen die Aerosol-Strömung in der Düse 10 auf Überschallgeschwindigkeit beschleunigt wird, wobei sich im sich erweiternden Austrittsabschnitt 12 der Düse 10 ein Mach-Schock - nicht dargestellt - ausbildet. Um die Aerosol-Strömung auf Überschallgeschwindigkeit zu beschleunigen und eine Ausbildung der Mach-Scheibe im Austrittsabschnitt 12 zu gewährleisten ist die beschriebene Formgebung der Düse 10 erforderlich.

Beim Durchtritt durch diese Mach-Schock-Zone werden die Gasmoleküle innerhalb einer Strecke von wenigen zehntel Millimeter stark von Überschallgeschwindigkeit auf Unterschallgeschwindigkeit verzögert. Die Pulverpartikel, die vor dem Mach-Schock langsamer als die Gasmoleküle waren, werden weniger stark verzögert als die Gasmoleküle, so daß nach dem Machschock die Pulverpartikel eine höhere Geschwindigkeit aufweisen als die Gasmoleküle. Hierdurch kommt es zu extrem hohen Reibungs- bzw. Scherkräften, welche an den Pulverteilchen angreifen und diese deagglomerieren.

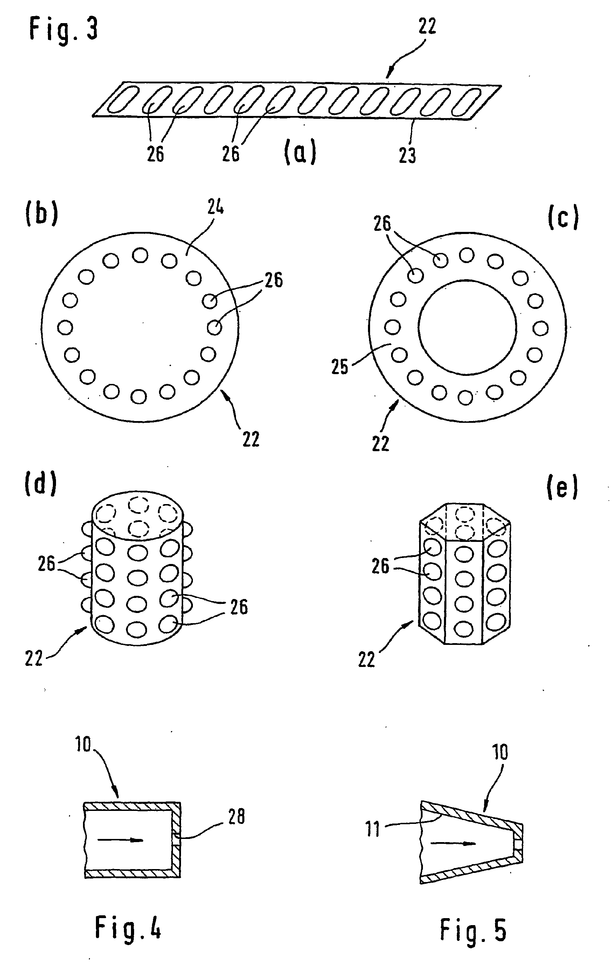

Fig.3a zeigt eine schematische Darstellung einer ersten Ausführungsform eines Mehrdosis-Blister-Behältnisses 22 zur Bevorratung der Pulver-Formulierung in der Seitenansicht. Das Mehrdosis-Blister-Behältnisse 22 ist linear als Blisterband 23 ausgebildet und verfügt über mehrere in einer Reihe angeordnete Kapsel 26, von denen jede Kapsel 26 eine Einzeldosis beinhaltet, wobei die jeweilige Kapsel 26 bei Gebrauch mittels einer geeigneten Vorrichtung (nicht dargestellt) geöffnet wird.

Fig.3b zeigt eine schematische Darstellung einer zweiten Ausführungsform eines Mehrdosis-Blister-Behältnisses 22 zur Bevorratung der Pulver-Formulierung in der Seitenansicht. Das Mehrdosis-Blister-Behältnis 22 ist flächig als Blister-Scheibe 24 ausgebildet und verfügt über eine Vielzahl von in einem Kreis angeordnete Kapseln 26.

Fig.3c zeigt eine schematische Darstellung einer dritten Ausführungsform eines Mehrdosis-Blister-Behältnisses 22 zur Bevorratung der Pulver-Formulierung in der Seitenansicht. Das Mehrdosis-Blister-Behältnis 22 ist flächig als Blister-Ring 25 ausgebildet und verfügt über eine Vielzahl von in einem Kreis angeordnete Kapseln 26.

Fig.3d zeigt eine schematische Darstellung einer vierten Ausführungsform eines Mehrdosis-Blister-Behältnisses 22 zur Bevorratung der Pulver-Formulierung in der perspektivischen Darstellung. Das Mehrdosis-Blister-Behältnis 22 ist dreidimensional als zylindrischer Körper ausgeführt. Derartige Mehrdosissysteme können 2 bis 90 Dosen enthalten, bevorzugt 5 bis 60 Dosen, insbesondere 7 bis 30 Dosen, wobei jede Dosis in einer separaten Kapsel 26 gelagert ist, die bei Gebrauch mittels einer geeigneten Vorrichtung geöffnet wird.

Fig.3e zeigt eine schematische Darstellung einer fünften Ausführungsform eines Mehrdosis-Blister-Behältnisses 22 zur Bevorratung der Pulver-Formulierung in der perspektivischen Darstellung. Das Mehrdosis-Blister-Behältnis 22 ist - wie in Fig.3d - dreidimensional als vielflächiger Körper ausgeführt.

Fig.4 zeigt eine schematische Darstellung einer Düse 10 einer dritten Ausführungsform eines Pulverinhalators in der Seitenansicht, geschnitten. Dabei handelt es sich um ein Lochblende 28, die weder über einen sich verjüngenden Eintrittsabschnitt noch über einen sich erweiternden Austrittsabschnitt verfügt.

Fig.5 zeigt eine schematische Darstellung einer Düse 10 einer vierten Ausführungsform eines Pulverinhalators in der Seitenansicht, geschnitten. Dabei handelt es sich um eine Lochblende 28, die über einen sich verjüngenden Eintrittsabschnitt 11, aber nicht über einen sich erweiternden Austrittsabschnitt verfügt.

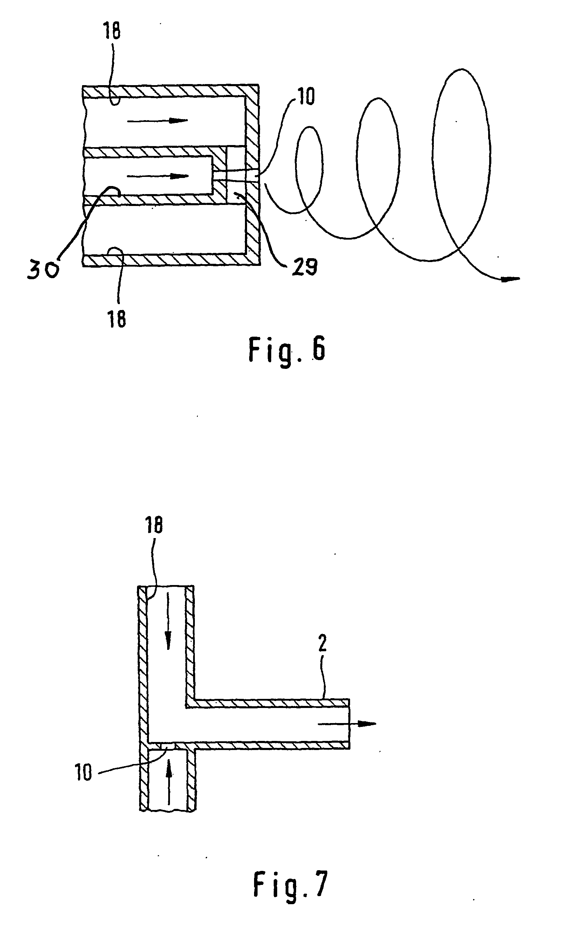

Fig.6 zeigt eine schematische Darstellung einer ersten Ausführungsform einer Vorrichtung zur Verzögerung der Aerosolströmung in der Seitenansicht, geschnitten. Beide Strömungen, sowohl die Einatmluftströmung im Einlasskanal 18 als auch die Aerosolströmung im Kanal 30, verlaufen zunächst parallel in zwei zueinander koaxialen Rohren, wobei die Einatmluft in eine wirbelförmige Strömung transformiert wird. Die in diese wirbelförmige Strömung injizierte Aerosolströmung weist eine verminderte Strömungsgeschwindigkeit auf.

Fig.7 zeigt eine schematische Darstellung einer zweiten Ausführungsform einer Vorrichtung zur Verzögerung der Aerosolströmung in der Seitenansicht, geschnitten. Die Einatemluftströmung und die Aerosolströmung werden aufeinander zugeleitet. Bei ihrem Zusammentreffen und infolge der Strömungsumlenkung wird die Vorwärtsgeschwindigkeit der Aerosolströmung vermindert.

### Bezugszeichenliste

- 1: Pulverinhalator
- 2: Mundstück
- 3: Druckmediumsystem
- 4: Ventil
- 5: Aktuator-Ventil
- 6: Vorrichtung zur Bevorratung
- 7: Pulver-Formulierung
- 8: Gasförmiges Druckmedium
- 9: Aerosol
- 10: Düse
- 11: Eintrittsabschnitt
- 12: Austrittsabschnitt
- 13: Mittelstück
- 14: Engster Querschnitt
- 15: Kapsel
- 16: Vorrichtung
- 17: Austrittsöffnung der Düse
- 18: Einlaßkanal
- 19: Durchflußsensor
- 20: Feder
- 21: Kolben
- 22: Mehrdosis-Blister-Behältnis
- 23: Blisterband
- 24: Blister-Scheibe
- 25: Blister-Ringe
- 26: Kapsel
- 27: Blisterkörper
- 28: Lochblende
- 29: Wirbelkammer
- 30: Kanal

## Patentansprüche

1. Pulverinhalator (1) , welcher über eine Hilfsenergiequelle in Form eines Druckmediumsystems (3) verfügt, mit einer Vorrichtung, welche einen pharmazeutischen Arzneistoff in Form einer Pulver-Formulierung (7) bevorratet. und bei Aktivierung des Druckmediumsystems ein von dem Druckmediumsystem (3) freigesetztes, gasförmiges Druckmedium (8) mit der Pulver-Formulierung (7) ein Aerosol (9) in der Art ausbildet, dass die Pulverpartikel in dem gasförmigen Druckmedium (8) dispergiert vorliegen, wobei in dem Inhalator (1) mit Mundstück (2) eine Düse (10) vorgesehen ist, **dadurch gekennzeichnet, dass** die Düse (10) durch das Aerosol (9) vor Verlassen des Inhalators (1) strömt, und das Druckmedium die Vorrichtung durchströmt, die den pharmazeutischen Arzneistoff in Form einer Pulver-Formulierung (7) bevorratet, um einen Teil der pharmazeutischen Arzneistoff in Form einer Pulver-Formulierung (7) in sich aufzunehmen, sodass nach Durchströmen der Vorrichtung zur Bevorratung (6) das gewünschte Aerosol in der Art vorliegt, dass die Pulverpartikel (7) in dem gasförmigen Druckmedium (8) dispergiert sind, das Aerosol 9) nachfolgend die Düse (10) und anschließend den Austrittsabschnitt (12) durchströmt.

2. Pulverinhalator (1) nach Anspruch 1, **dadurch gekennzeichnet, daß** die Düse (10) als Lochblende ausgebildet ist,

3. Pulverinhalator (1) nach Anspruch 1, **dadurch gekennzeichnet, daß** die Düse (10) einen sich verjüngenden Eintrittsabschnitt (11) aufweist, dem sich eine Lochblende anschließt.

4. Pulverinhalator (1) nach Anspruch 1 , **dadurch gekennzeichnet, daß** die Düse (10) einen sich verjüngenden Eintrittsabschnitt (11), ein Mittelstück (13) und einen sich erweiternden Austrittsabschnitt (12) aufweist, der sich an das Mittelstück (13) anschließt.

5. Pulverinhalator nach Anspruch 4, **dadurch gekennzeichnet, daß** in dem Mittelstück (13) der engste Düsenquerschnitt angeordnet ist.

6. Pulverinhalator (1) nach Anspruch 5, **dadurch gekennzeichnet, daß** die Düse (10) eine Lavaldüse ist.

7. Pulverinhalator (1) nach Anspruch 4 bis 6, **dadurch gekennzeichnet, daß** der engste Querschnitt (14) der Düse (10) 100µm bis 1500µm, vorzugsweise 400µm bis 800µm, im Durchmesser beträgt.

8. Pulverinhalator (1) nach einem der Ansprüche 4 bis 7, **dadurch gekennzeichnet, daß** die Düse (10) einen erweiternden Austrittsabschnitt (12) in der Art aufweist, daß im Austrittsabschnitt (12) das Druckmedium (8) auf Überschallgeschwindigkeit beschleunigt wird.

9. Pulverinhalator (1) nach einem der vorherigen Ansprüche, **dadurch gekennzeichnet, daß** das Druckmediumsystem (3) eine mit der Umgebung in Verbindung stehende Pumpe aufweist und die Umgebungsluft als Druckmedium (8) verwendet.

10. Pulverinhalator (1) nach einem der Ansprüche 1 bis 8, **dadurch gekennzeichnet, daß** das Druckmediumsystem (3) eine Patrone umfaßt, welche ein unter Druck stehendes Druckmedium (8) bevorratet.

11. Pulverinhalator (1) nach einem der vorherigen Ansprüche, **dadurch gekennzeichnet, daß** Luft als Druckmedium (8) vorgesehen wird.

12. Pulverinhalator (1) nach einem der Ansprüche 1 bis 8 oder 10 bis 11, **dadurch gekennzeichnet, daß** N₂, CO₂, Ar oder He als Druckmedium (8) vorgesehen wird.

13. Pulverinhalator (1) nach einem der vorherigen Ansprüche, **dadurch gekennzeichnet, daß** die Vorrichtung zur Bevorratung (6) der Pulver-Formulierung (7) zwischen dem Druckmediumsystem (3) und Düse (10) angeordnet ist in der Art, daß das Druckmedium (8) die Vorrichtung (6) passieren muß.

14. Pulverinhalator (1) nach einem der vorherigen Ansprüche, **dadurch gekennzeichnet, daß** die Vorrichtung zur Bevorratung (6) der Pulver-Formulierung (7) eine mit Pulver (7) gefüllte Kapsel (15) aufweist.

15. Pulverinhalator (1) nach Anspruch 14, **dadurch gekennzeichnet, daß** die Kapsel (15) als Verbrauchsmaterial austauschbar ist.

16. Pulverinhalator (1) nach einem der Ansprüche 1 bis 13, **dadurch gekennzeichnet, daß** die Vorrichtung zur Bevorratung (6) der Pulver-Formulierung (7) ein Mehrdosis-Blister-Behältnis umfaßt.

17. Pulverinhalator (1) nach einem der Ansprüche 1 bis 8 oder 10 bis 16, **dadurch gekennzeichnet, daß** im Mundstück (2) ein Durchfluß-Sensor (19) vorgesehen ist, der ein Eingangssignal für das Druckmediumsystem (3) generiert.

18. Pulverinhalator (1) nach einem der vorhergehenden Ansprüche, **dadurch gekennzeichnet, daß** zwischen dem Austrittsabschnitt (12) und dem Austritt des Mundstückes (2) eine wirbelförmige Strömung der durch ein Einlaßkanal angesogenen Einatemluft erzeugt wird.

19. Pulverinhalator (1) nach einem der vorhergehenden Ansprüche, **dadurch gekennzeichnet, daß** die Düse(10) und ein Einlaßkanal (18) für die Einatemluft so angeordnet sind, dass die aus der Düse (10) austretende Aerosolströmung und die Einatemluft entgegengerichtet sind (Fig. 7)

20. Pulverinhalator (1) nach einem der Ansprüche 1 bis 19, **dadurch gekennzeichnet, daß** die Düse (10) und ein Einlasskanal für die Einatemluft so angeordnet sind, dass die aus der Düse austretende Aerosolströmung und die Einatemluft in einem Winkel aufeinander prallen.

21. Pulverinhalator (1) nach einem der Ansprüche 1 bis 20, **dadurch gekennzeichnet, dass** der die Aerosolströmung führende Kanal (30) und die Einlaßkanäle(18) für die Einatemluft in einer Wirbelkammer (29) münden, von der aus die Aerosolwolke zur Düse (10) geleitet wird (Fig. 6)

## Claims

1. Powder inhaler (1) which comprises an auxiliary energy source in the form of a pressure medium system (3), having a device which supplies a pharmaceutical medicament in the form of a powder formulation (7), and on activation of the pressure medium system a gaseous pressure medium (8) released by the pressure medium system (3) forms an aerosol (9) with the powder formulation (7) such that the powder particles are present in the gaseous pressure medium (8) in dispersed form, wherein a nozzle (10) is provided in the inhaler (1) with mouthpiece (2), **characterised in that** the aerosol (9) flows through the nozzle (9) before leaving the inhaler (1), and the pressure medium flows through the device which supplies the pharmaceutical medicament in the form of a powder formulation (7), in order to take up into itself a proportion of the pharmaceutical medicament in the form of a powder formulation (7), so that after flowing through the supply device (6) the desired aerosol is present such that the powder particles (7) are in dispersed form In the gaseous pressure medium (8), after which the aerosol (9) flows through the nozzle (10) and then through the exit section (12).

2. Powder inhaler (1) according to claim 1, **characterised in that** the nozzle (10) is in the form of a perforated screen.

3. Powder inhaler (1) according to claim 1, **characterised in that** the nozzle (10) has a tapering entry section (11) adjoining which is a perforated screen.

4. Powder inhaler (1) according to claim 1, **characterised in that** the nozzle (10) has a tapering entry section (11), middle part (13) and a flaring exit section (12) which is adjacent to the middle part (13).

5. Powder inhaler according to claim 4, **characterised in that** the narrowest cross-section is arranged in the middle part (13).

6. Powder Inhaler (1) according to claim 5, **characterised in that** the nozzle (10) is a Laval nozzle.

7. Powder inhaler (1) according to claims 4 to 6, **characterised in that** the narrowest cross-section (14) of the nozzle (10) is 100 µm to 1500 µm, preferably 400 µm to 800 µm, in diameter.

8. Powder inhaler (1) according to one of claims 4 to 7, **characterised in that** the nozzle (10) has a flaring exit section (12) such that in the exit section (12) the pressure medium (8) is accelerated to supersonic speed.

9. Powder inhaler (1) according to one of the preceding claims, **characterised in that** the pressure medium system (3) comprises a pump connected to the environment and uses the ambient air as pressure medium (8).

10. Powder inhaler (1) according to one of claims 1 to 8, **characterised in that** the pressure medium system (3) comprises a cartridge which supplies a pressure medium (8) under pressure.

11. Powder inhaler (1) according to one of the preceding claims, **characterised in that** air is provided as the pressure medium (8).

12. Powder inhaler (1) according to one of claims 1 to 8 or 10 to 11. **characterised in that** N₂, CO₂, Ar or He is provided as the pressure medium (8).

13. Powder inhaler (1) according to one of the preceding claims, **characterised in that** the device for supplying (6) the powder formulation (7) is arranged between the pressure medium system (3) and nozzle (10) such that the pressure medium (8) has to pass the device (6).

14. Powder inhaler (1) according to one of the preceding claims, **characterised in that** the device for supplying (6) the powder formulation (7) comprises a capsule (15) filled with Powder (7).

15. Powder inhaler (1) according to claim 14, **characterised in that** the capsule (15) is replaceable as a consumable material.

16. Powder inhaler (1) according to one of claims 1 to 13, **characterised in that** the device for supplying (6) the powder formulation (7) comprises a multi-dose blister container.

17. Powder inhaler (1) according to one of claims 1 to 8 or 10 to 16, **characterised in that** a throughflow sensor (19) is provided in the mouthpiece (2), which generates an input signal for the pressure medium system (3).

18. Powder inhaler (1) according to one of the preceding claims, **characterised in that** between the exit section (12) and the exit of the mouthpiece (2) a turbulent flow is produced in the inspired air sucked in through an inlet channel.

19. Powder inhaler (1) according to one of the preceding claims, **characterised in that** the nozzle (10) and an inlet channel (18) for the inspired air are arranged so that the aerosol flow emerging from the nozzle (10) and the inspired air are directed counter to one another (Fig. 7).

20. Powder inhaler (1) according to one of claims 1 to 19, **characterised in that** the nozzle (10) and an inlet channel for the inspired air are arranged so that the aerosol flow leaving the nozzle and the inspired air strike one another at an angle.

21. Powder inhaler (1) according to one of claims 1 to 20, **characterised in that** the channel (30) carrying the aerosol flow and the inlet channels (18) for the inspired air open into a turbulence chamber (29) from which the aerosol cloud is conveyed to the nozzle (10) (Fig. 6).

## Revendications

1. Inhalateur de poudre (1) qui dispose d'une source d'énergie auxiliaire sous la forme d'un système de milieu de pression (3) comportant un dispositif qui renferme une substance médicamenteuse pharmaceutique sous la forme d'une formulation en poudre (7) et lors de l'activation du système de milieu de pression, un milieu de pression (8) sous forme de gaz, libéré du système de milieu de pression constitue avec la formulation en poudre (7) un aérosol (9) de telle sorte que les particules de poudre soient dispersées dans le milieu de pression (8) sous forme gazeuse, une buse (10) étant prévue dans l'inhalateur (1) avec un embout buccal (2), **caractérisé en ce que** l'aérosol (9) s'écoule par la buse (10) avant de quitter l'inhalateur (1) et le milieu de pression passe par le dispositif qui renferme la substance médicamenteuse pharmaceutique sous la forme d'une formulation en poudre (7) pour absorber dans ledit milieu une partie de la substance médicamenteuse pharmaceutique sous la forme d'une formulation en poudre (7) de sorte qu'après le passage par le dispositif de stockage (6), l'aérosol souhaité se présente de telle sorte que les particules de poudre (7) soient dispersées dans le milieu de pression sous forme de gaz (8), l'aérosol (9) passe ensuite par la buse (10) et enfin, dans le segment de sortie (12).

2. Inhalateur de poudre (1) selon la revendication 1, **caractérisé en ce que** la buse (10) est constituée comme un obturateur perforé.

3. Inhalateur de poudre (1) selon la revendication 1, **caractérisé en ce que** la buse (10) présente un segment d'entrée s'affinant, auquel est adjoint un obturateur perforé.

4. Inhalateur de poudre (1) selon la revendication 1, **caractérisé en ce que** la buse (10) présente un segment d'entrée s'affinant (11), une pièce intermédiaire (13) et un segment de sortie (12) s'élargissant, auquel est raccordée la pièce intermédiaire (13).

5. Inhalateur de poudre selon la revendication 4, **caractérisé en ce que** dans la pièce intermédiaire (13), est disposé le segment de buse le plus étroit.

6. Inhalateur de poudre (1) selon la revendication 5, **caractérisé en ce que** la buse (10) est une buse convergente-divergente.

7. Inhalateur de poudre (1) selon la revendication 4 à 6, **caractérisé en ce que** le segment le plus étroit (14) de la buse (10) a un diamètre de 100 µm à 1500 µm, de préférence, de 400 µm à 800 µm.

8. Inhalateur de poudre (1) selon l'une des revendications 4 à 7, **caractérisé en ce que** la buse (10) présente un segment de sortie s'élargissant (12) de telle sorte que dans le segment de sortie, le milieu de pression (8) soit accéléré à une vitesse supersonique.

9. Inhalateur de poudre (1) selon l'une des revendications précédentes, **caractérisé en ce que** le système de milieu de pression (3) présente une pompe en communication avec l'environnement et utilise l'air ambiant comme milieu de pression (8).

10. Inhalateur de poudre (1) selon l'une des revendications 1 à 8, **caractérisé en ce que** le système de milieu de pression (3) comprend une cartouche qui renferme un milieu de pression sous pression.

11. Inhalateur de poudre (11) selon l'une des revendications précédentes, **caractérisé en ce que** de l'air est prévu comme milieu de pression (8).

12. Inhalateur de poudre (1) selon l'une des revendications 1 à 8 ou 10 à 11, **caractérisé en ce que** du N₂, CO₂, Ar ou He sont prévus comme milieu de pression (8).

13. Inhalateur de poudre (1) selon l'une des revendications précédentes, **caractérisé en ce que** le dispositif est disposé pour le stockage (6) de la formulation en poudre (7) entre le système de milieu de pression (3) et la buse (10) de telle sorte que le milieu de pression (8) doive passer dans le dispositif (6).

14. Inhalateur de poudre (1) selon l'une des revendications précédentes, **caractérisé en ce que** le dispositif présente, pour le stockage (6) de la formulation en poudre (7), une capsule (15) remplie de poudre (7).

15. Inhalateur de poudre (1) selon la revendication 14, **caractérisé en ce que** la capsule (15) est échangeable en tant que matériel consommable.

16. Inhalateur de poudre (1) selon l'une des revendications 1 à 13, **caractérisé en ce que** le dispositif comprend pour le stockage (6) de la formulation en poudre (7), un conteneur multidose sous blister.

17. Inhalateur de poudre (1) selon l'une des revendications 1 à 8 ou 10 à 16, **caractérisé en ce que** dans l'embout buccal (2), un capteur de débit (19) est prévu, qui génère un signal d'entrée du système de milieu de pression (3).

18. Inhalateur de poudre (1) selon l'une des revendications précédentes, **caractérisé en ce que**, entre le segment de sortie (12) et la sortie de l'embout buccal (2) est produit un courant tourbillonnant de l'air inspiré, aspiré par un canal d'admission.

19. Inhalateur de poudre (1) selon l'une des revendications précédentes, **caractérisé en ce que** la buse (10) et un canal d'admission (18) pour l'air inspiré sont disposés de telle sorte que le flux d'aérosol sortant de la buse (10) et l'air inspiré soient dirigés l'un contre l'autre (figure 7).

20. Inhalateur de poudre (1) selon l'une des revendications 1 à 19, **caractérisé en ce que** la buse (10) et un canal d'admission pour l'air inspiré sont disposés de telle sorte que le flux d'aérosol sortant de la buse et l'air inspiré se heurtent dans un angle.

21. Inhalateur de poudre (1) selon l'une des revendications 1 à 20, **caractérisé en ce que** le canal (30) conduisant le flux d'aérosol (30) et les canaux d'admission (18) pour l'air inspiré débouchent dans une chambre à remous (29) de laquelle le nuage d'aérosol est conduit vers la buse (10) (figure 6).
